# EUROPEAN PATENT APPLICATION

(11) **EP 4 656 735 A1**
(43) Date of publication of application: **03.12.2025**
(21) Application number: 24747253.3
(22) Date of filing: 22.01.2024
(51) Int. Cl.: C12Q 1/6848, C12Q 1/686, C12Q 1/6851, C12Q 1/6869

(54) **INIBHITION OF REVERSE TRANSCRIPTION DERIVED FROM RIBOSOMAL RNA**

(30) Priority: 26.01.2023 JP 2023010502
(71) Applicant: Toyobo Co., Ltd., Kita-ku Osaka-shi, Osaka 530-0001 (JP); RIKEN, Wako-shi, Saitama 351-0198 (JP)
(72) Inventor: TAKEDA, Taka-aki, Tsuruga-shi, Fukui 914-8550 (JP); HAYASHI, Tetsutaro, Wako-shi, Saitama 351-0198 (JP); NIKAIDO, Itoshi, Wako-shi, Saitama 351-0198 (JP)
(74) Representative: Müller-Boré & Partner Patentanwälte PartG mbB
(86) International application number: PCT/JP2024/001631
(87) International publication number: WO 2024/157925

(57) **Abstract**

Provided are a composition and a method for suppressing the reverse transcription of a ribosomal RNA. The composition for suppressing the reverse transcription of a ribosomal RNA according to the present invention has features such that the composition comprises a polymer having at least one functional group selected from the group consisting of a sulfuric acid group, a sulfonic group, and salt-form groups thereof, and that the composition is used in an amount such that a reverse transcription reaction solution contains the polymer in a concentration of 10 pg/µL or more.

## Description

### Technical Field

The present invention relates to the production and amplification of nucleic acids in which reverse transcription derived from ribosomal RNA is suppressed. The present invention relates to, for example, a composition and method useful in producing and amplifying a nucleic acid from a ribonucleic acid (RNA) template, specifically, a composition and method for producing and amplifying a nucleic acid by a reverse transcription reaction using a specific polymer. The composition and the method according to the present invention are used in, for example, suppressing the reverse transcription of ribosomal RNA, more specifically, suppressing the synthesis of DNA derived from ribosomal RNA.

### Background Art

Ribosomal RNA is considered to account for about 80 to 90% of RNA in vivo. In the analysis of gene expression levels, it is common to convert expressed RNA into cDNA by a reverse transcription reaction and perform gene analysis based on the cDNA. If ribosomal RNA, which makes up the majority of RNA in vivo, is reverse-transcribed and amplified during this reverse transcription reaction, noise is generated in the expression analysis of RNA that should be reverse-transcribed and amplified. Further, in recent years, new technologies, such as an RT-RamDA method, that enable the generation of a large amount of amplified nucleic acid products by using RNA as a template, have been developed (see Patent Literature (PTL) 1). In these techniques, such as the RT-RamDA method, which is capable of generating a large amount of amplified nucleic acid products using RNA as a template, once the reverse transcription reaction of ribosomal RNA occurs, ribosomal RNA is isothermally amplified by a strand displacement reaction. This greatly affects the analysis, reducing the relative amount of RNA that should be analyzed. Thus, it is desirable to suppress the synthesis of DNA derived from ribosomal RNA in nucleic acid analysis methods that comprise performing a reverse transcription reaction.

Until now, a method has been investigated in which a specific nucleic acid polymer is used to suppress reverse transcription of ribosomal RNA in a reverse transcription reaction from a template RNA (Patent Literature (PTL) 2). However, the development of a more useful method for suppressing reverse transcription from ribosomal RNA is desired.

### Citation List

### Patent Literature

PTL 1: WO2016/052619
PTL 2: WO2020/184551

### Summary of Invention

### Technical Problem

An object of the present invention is to provide a means for suppressing the reverse transcription of ribosomal RNA.

### Solution to Problem

The present inventors conducted extensive research to achieve the object and found the following. In a method of synthesizing DNA by reverse transcription of template RNA, the use of a polymer having at least one functional group selected from the group consisting of a sulfuric acid group (-O-SO₃H), a sulfonic group (-SO₃H), and salt-form groups thereof at a specific concentration or higher can suppress the reverse transcription of ribosomal RNA and suppress the synthesis of DNA derived from ribosomal RNA. The present inventors conducted further research based on this finding and accomplished the present invention.

Specifically, the present invention includes the following subject matter.

### Item 1.

A method for synthesizing DNA by reverse transcription of template RNA, the method comprising suppressing synthesis of DNA derived from ribosomal RNA using a polymer comprising at least one functional group selected from the group consisting of a sulfuric acid group, a sulfonic group, and salt-form groups thereof wherein the polymer is present in a concentration of 10 pg/µL or more in a reverse transcription reaction solution.

### Item 2.

The method according to Item 1, wherein the polymer comprises at least one member selected from the group consisting of sulfated glycosaminoglycan, polyvinyl sulfonic acid, and salts thereof.

### Item 3.

The method according to Item 1 or 2, wherein the polymer comprises at least one member selected from the group consisting of heparin, heparan sulfate, chondroitin sulfate, and salts thereof.

### Item 4.

The method according to any one of Items 1 to 3, wherein the reverse transcription reaction solution contains the polymer in a concentration of 10 pg/µL or more and 1 µg/µL or less.

### Item 5.

The method according to any one of Items 1 to 4, wherein the reverse transcription is performed by using a random primer.

### Item 6.

The method according to Item 5, wherein the random primer has a base sequence that is not completely complementary to the base sequence of the ribosomal RNA.

### Item 7.

The method according to any one of Items 1 to 6, wherein the reverse transcription is performed in an RT-PCR method or RT-RamDA method.

### Item 8.

The method according to any one of Items 1 to 7, wherein the template RNA is RNA extracted from a cell.

### Item 9.

A composition for suppressing reverse transcription of ribosomal RNA,
the composition comprising a polymer having at least one functional group selected from the group consisting of a sulfuric acid group, a sulfonic group, and salt-form groups thereof,
the composition being used in an amount such that a reverse transcription reaction solution contains the polymer in a concentration of 10 pg/µL or more.

### Item 10.

A reverse transcription reaction composition for preparing a reverse transcription reaction product for use in next-generation sequencing, the reverse transcription reaction composition comprising a polymer having at least one functional group selected from the group consisting of a sulfuric acid group, a sulfonic group, and salt-form groups thereof in an amount of 10 pg/µL or more.

### Item 11.

The composition according to Item 9 or 10, wherein the reverse transcription is performed in an RT-PCR method or an RT-RamDA method.

### Item 12.

The composition according to any one of Items 9 to 11, which is in the form of a kit.

### Advantageous Effects of Invention

According to the present invention, the reverse transcription of ribosomal RNA or the synthesis of DNA derived from ribosomal RNA can be suppressed.

### Description of Embodiments

### Method for Suppressing the Synthesis of DNA Derived from

### Ribosomal RNA (rRNA) in a Method of Synthesizing DNA by Reverse Transcription of Template RNA

### 1. Suppression of Synthesis of DNA derived from Abundant rRNA by a Polymer Having at Least One Functional Group Selected from the Group Consisting of a Sulfuric Acid Group, a Sulfonic Group, and Salt-form Groups Thereof

In the present invention, reverse transcription from abundant rRNA can be suppressed by allowing a template RNA, a reverse transcriptase, and a polymer having at least one functional group selected from the group consisting of a sulfuric acid group, a sulfonic group, and salt-form groups thereof to be present together during a reverse transcription reaction. This can increase the proportion of DNA derived from the target RNA in the product after the reverse transcription reaction. While not wishing to be bound by theory, it is considered that a polymer having at least one functional group selected from the group consisting of a sulfuric acid group, a sulfonic group, and salt-form groups thereof competitively inhibits the binding of a primer to rRNA and that at least partially based on this property, the polymer inhibits the reverse transcription of abundant rRNA by reverse transcriptase.

The polymer having at least one functional group selected from the group consisting of a sulfuric acid group, a sulfonic group (also referred to as a "sulfonic acid group"), and salt-form groups thereof is not particularly limited as long as it has a side chain of the functional group in its backbone. Examples of salt-form groups of a sulfuric acid group and/or a sulfonic group (a sulfuric acid salt group and/or a sulfonic acid salt group) include -O-SO₃-M⁺ and/or -SO₃-M⁺ (wherein M⁺ is an alkali metal ion, such as a sodium ion or a potassium ion, or NR₄⁺, wherein each R independently represents H or an optionally substituted hydrocarbon group, and two or three R's together with N adjacent thereto may form a ring). Further, examples of polymers having a sulfuric acid salt group and/or a sulfonic acid salt group (salts of polymers having a sulfuric acid group and/or a sulfonic group) include polymers in which -O-SO₃⁻ and/or -SO₃⁻ in the side chain is crosslinked by a polyvalent cation (e.g., an alkaline earth metal ion, such as a calcium ion). In one embodiment, the polymer having at least one functional group selected from the group consisting of a sulfuric acid group, a sulfonic group, and salt-form groups thereof preferably contains at least one polymer selected from the group consisting of polymers having a sulfuric acid group and/or a salt-form group thereof and polymers having a sulfonic group and/or a salt-form group thereof, and more preferably contains at least one polymer selected from the group consisting of sulfated glycosaminoglycan, polyvinyl sulfonic acid, derivatives thereof, and salts thereof. These polymers that can be used may be synthetically produced, extracted from natural products, or commercially available.

The sulfated glycosaminoglycan that can be used is not particularly limited, and can be of any type. The glycosaminoglycan is preferably a polysaccharide that has a repeating unit of a disaccharide of an amino sugar and either uronic acid or galactose. Examples of amino sugars include glucosamine and galactosamine. The amino sugar may be N-acetylated amino sugar. Examples of uronic acids include glucuronic acid, iduronic acid, and galacturonic acid. The sulfated glycosaminoglycan is preferably a glycosaminoglycan having 1, 2, 3, or 4 sulfuric acid groups per repeating unit of the disaccharide. The sulfuric acid group is preferably present as a substituent for a hydroxyl group and/or an amino group on an amino sugar and/or uronic acid or galactose. Examples of sulfated glycosaminoglycans include those shown in the table below.

| | | Amino sugar-uronic acid |
|---|---|---|
| Heparin | | GlcNAc-GlcA |
| | | GlcNS-GlcA |
| | | GlcNS-IdoA |
| | | GlcNS-IdoA(2S) |
| | | GlcNS(6S)-IdoA |
| | | GlcNS(6S)-IdoA(2S) |
| Heparan sulfate | | GlcNAc-GlcA |
| | | GlcNS-GlcA |
| | | GlcNS-IdoA |
| | | GlcNS-IdoA(2S) |
| | | GlcNS(6S)-IdoA |
| | | GlcNS(6S)-IdoA(2S) |
| Keratan sulfate | | GlcNS(6S)-Gal |
| Chondroitin sulfate | A | GlcNAc(4S)-GlcA |
| | B (Dermatan sulfate) | GlcNAc(4S)-IdoA(2S) |
| | C | GlcNAc(6S)-GlcA |
| | D | GlcNAc(6S)-GlcA(2S) |
| | E | GIcNAc(4S, 6S)-GIcA |
| | etc. | |
| Amino sugar | GlcNAc : D-Glucosamine (2-N-acetylated) | |
| | GlcNAc(4S) : D-Glucosamine (2-N-acetylated, 4-O-sulfated) | |
| | GlcNAc(6S) : D-Glucosamine (2-N-acetylated, 4-O-sulfated) | |
| | GlcNAc(4S, 6S) : D-Glucosamine (2-N-acetylated, 4-O-sulfated, 6-O-sulfated) | |
| | GlcNS : D-Glucosamine (2-N-sulfated) | |
| | GlcNS(6S) : D-Glucosamine (2-N-sulfated, 6-O-sulfated) | |
| Uronic acid | GlcA : D-glucuronic acid | |
| | GlcA(2S) : D-Glucuronic acid (2-O-sulfated) | |
| | IdoA : L-Iduronic Acid | |
| | IdoA(2S) : L-Iduronic acid (2-O-sulfated) | |
| Galactose | Gal : D-Galactose | |

The sulfated glycosaminoglycan and/or a salt thereof can bind to a protein to constitute a proteoglycan. As long as the effect of the present invention is provided, any compound that has a structure such as the structure of proteoglycan can be used as a glycosaminoglycan that has a sulfuric acid group and/or a salt-form group thereof.

From the standpoint of more effectively suppressing the reverse transcription of rRNA or the synthesis of DNA derived from rRNA, sulfated glycosaminoglycan and/or a salt thereof preferably comprises at least one member selected from the group consisting of heparin, heparan sulfate, keratan sulfate, chondroitin sulfate, derivatives thereof, and salts thereof, more preferably comprises at least one member selected from the group consisting of heparin, chondroitin sulfate, derivatives thereof, and salts thereof, and even more preferably comprises at least one member selected from the group consisting of heparin, chondroitin sulfate C, derivatives thereof, and salts thereof.

Polyvinyl sulfonic acid that can be used is not particularly limited as long as it is a polymer containing vinyl sulfonic acid (or ethylene sulfonic acid) as a constituent unit, and can be of any kind. The polyvinyl sulfonic acid may be a homopolymer of vinyl sulfonic acid or a copolymer comprising a structural unit other than vinyl sulfonic acid (e.g., a structural unit of (meth)acrylic acid, (meth)acrylic acid ester, (meth)acrylic acid amide, (meth)acrylonitrile, or the like). The polyvinyl sulfonic acid is preferably a polymer comprising 50 mole% or more of a structural unit of vinyl sulfonic acid, more preferably a polymer comprising 70 mole% or more of a structural unit of vinyl sulfonic acid, even more preferably a polymer comprising 90 mole% or more of a structural unit of vinyl sulfonic acid, and still even more preferably a homopolymer of vinyl sulfonic acid.

The "derivatives" refer to polymers that are partly modified in structure while maintaining the original polymer backbone. Examples of modification include oxidation, reduction, replacement of an atom, and introduction of a functional group.

The "salts" are not particularly limited, and preferable examples include amine salts, alkali metal salts, and alkaline earth metal salts. Amine salts include salts having a cation represented by NR₄⁺ (wherein each R independently represents H or an optionally substituted hydrocarbon group, and two or three R's together with N adjacent thereto may form a ring). Examples of such salts include ammonium salts, monomethylammonium salts, monoethylammonium salts, dimethylammonium salts, diethylammonium salts, trimethylammonium salts, triethylammonium salts, tetramethylammonium salts, and pyridinium salts. Preferable examples of the "salts" include ammonium salts, sodium salts, potassium salts, and calcium salts. Usable salts may also include hydrate salts.

The weight average molecular weight of the polymer having at least one functional group selected from the group consisting of a sulfuric acid group, a sulfonic group, and salt-form groups thereof is not particularly limited. The polymer has a weight average molecular weight of, for example, 5,000 or more, preferably 10,000 or more, more preferably 20,000, and can be 50,000 or more, or 100,000 or more. The upper limit of the weight average molecular weight of the polymer is not particularly limited as long as the effect of the present invention is provided. The upper limit is, for example, 5,000,000 or less, and preferably 1,000,000 or less, and can be 500,000 or less.

The weight average molecular weight can be measured by a usual method using gel permeation chromatography (GPC) and calculated as a pullulan converted weight average molecular weight from a standard pullulan calibration curve that has been prepared in advance (see, for example, US2020/190266A, the entire content of which is incorporated herein by reference).

The number (or the average number) of repetitions of the disaccharide structural unit of an amino sugar and uronic acid or galactose in sulfated glycosaminoglycan, or the number (or the average number) of repetitions of a vinyl sulfonic acid (or ethylene sulfonic acid) structural unit contained in polyvinyl sulfonic acid is not particularly limited and can be, for example, 20 or more, preferably 30 or more, more preferably 40 or more, and even more preferably 50 or more, and may be, for example, 100 or more, or 150 or more. The number (or the average number) of repetitions of the structural unit can be, for example, 5,000 or less, 4,000 or less, 3,000 or less, 1,000 or less, or 500 or less. The repetition of the structural unit may be continuous or discontinuous, preferably continuous.

### 2. Method of Synthesizing DNA by Reverse Transcription of Template RNA

The method of synthesizing DNA by reverse transcription of template RNA is not particularly limited, and various known methods can be used. The method typically comprises the step of incubating a reverse transcription reaction composition (or a reverse transcription reaction solution) containing a protein having reverse transcription activity, such as reverse transcriptase.

### 2-1. Reverse Transcription Reaction Composition

The reverse transcription reaction composition contains, for example, a polymer having at least one functional group selected from the group consisting of a sulfuric acid group, a sulfonic group, and salt-form groups thereof, a template RNA, a primer, a deoxyribonucleotide, and a reverse transcriptase. Further, the reverse transcription reaction composition may optionally contain other components, such as a DNA polymerase.

From the standpoint of suppressing the reverse transcription of rRNA and suppressing the synthesis of DNA derived from rRNA, the concentration of the polymer in the reverse transcription reaction composition can be, for example, 10 pg/µL or more, preferably 15 pg/µL, 20 pg/µL, 50 pg/µL or more, 100 pg/µL or more, 1 ng/µL or more, or 10 ng/µL or more. Further, from the standpoint of not excessively suppressing the synthesis of DNA derived from the target RNA other than rRNA, the concentration of the polymer is, for example, 1 µg/µL or less, preferably 100 ng/µL or less, more preferably 20 ng/µL or less, and can be, for example, 10 ng/µL or less, 5 ng/µL or less, 3 ng/µL or less, 2.5 ng/µL or less, 2 ng/µL or less, 1 ng/µL or less, or less than 1 ng/µL.

In one embodiment, when sulfated glycosaminoglycan and/or a salt thereof is used as the polymer having at least one functional group selected from the group consisting of a sulfuric acid group, a sulfonic group, and salt-form groups thereof, the concentration of the polymer in the reverse transcription reaction solution is preferably adjusted to be relatively low, and can be, for example, 2 ng/µL or less, preferably 1 ng/µL or less, 500 pg/µL or less, 200 pg/µL or less, 100 pg/µL or less, or 50 pg/µL or less. The lower limit of the concentration of the polymer in the reverse transcription reaction solution is not particularly limited, but is preferably adjusted to, for example, 10 pg/µL or 15 pg/µL or more.

In another embodiment, when polyvinyl sulfonic acid and/or a salt thereof is used as the polymer having at least one functional group selected from the group consisting of a sulfuric acid group, a sulfonic group, and salt-form groups thereof, the concentration of the polymer in the reverse transcription reaction solution is preferably adjusted to be relatively high, and can be, for example, 100 pg/µL or more, preferably 200 pg/µL or more or 500 pg/µL or more, more preferably 1 ng/µL or more, 2 ng/µL or more, or 10 ng/µL or more. The upper limit of the concentration of the polymer in the reverse transcription reaction solution is not particularly limited, and is preferably adjusted to, for example, 100 ng/µL or less.

### (1) Template RNA

The template RNA can be any RNA such as RNA extracted from tissue or cells, or RNA extracted from tissue or cells and then further purified (e.g., purified RNA treated with a purification technique known in the art, such as ethanol precipitation or column purification). In particular, the template RNA for use is preferably RNA extracted from cells. Typically, RNA extracted from cells inevitably contains rRNA in addition to RNA for use as template RNA such as mRNA. However, because the reverse transcription of rRNA is suppressed in the presence of the polymer having at least one functional group selected from the group consisting of a sulfuric acid group, a sulfonic group, and salt-form groups thereof, template RNA can be specifically reverse-transcribed even by using such RNA extracted from cells as template RNA. More specifically, the step of removing rRNA from RNA extracted from cells can be eliminated.

The cells from which RNA is extracted are not particularly limited in type, and can be of any type. The number of cells can be suitably adjusted with a cell sorter. For example, RNA extracted from a small number of cells (e.g., 1 to 100 cells, preferably 1 to 10 cells, more preferably 1 or 2 cells, and more preferably 1 cell) can be used.

The method for extracting RNA from cells typically includes the step of lysing cells by using a composition for cell lysis that contains a cell lysis reagent.

Examples of cell lysis reagents include surfactants and chaotropic agents. Examples of surfactants include anionic surfactants (e.g., sodium dodecyl sulfate, sodium cholate, and sodium deoxycholate), cationic surfactants (e.g., cetyltrimethylammonium bromide), nonionic surfactants (e.g., octylphenol ethoxylate, polyoxyethylene lauryl ether, polyoxyethylene stearyl ether, and polyoxyethylene sorbitan monolaurate), and zwitterionic surfactants (e.g., 3-[(3-cholamidopropyl)dimethylammonio]-1-propanesulfonic acid). Examples of chaotropic agents include urea and lithium salts such as lithium perchlorate. The cell lysis reagent is typically dissolved in water (preferably nuclease-free water) and used in the form of an aqueous solution.

The composition for cell lysis may contain a protease (e.g., protease K), an RNase inhibitor, or a combination of two or more of these components.

The composition for cell lysis may or may not contain a polymer having at least one functional group selected from the group consisting of a sulfuric acid group, a sulfonic group, and salt-form groups thereof. When RNA extracted from cells by using a composition for cell lysis that contains the above polymer is used as template RNA, the above polymer does not need to be added to the reverse transcription reaction composition.

The details of the composition for cell lysis are disclosed, for example, in US Patent Application Publication No. 2011/0111463, the entire disclosure of which is incorporated herein by reference.

The concentration of RNA (including rRNA) in the reverse transcription reaction composition is, for example, 1 to 10000 pg/µL, preferably 10 to 1000 pg/µL, or more preferably 1 to 100 pg/µL.

### (2) Primer

The primer includes a primer specific to template RNA, an oligo-dT primer, a random primer, and a combination of two or more thereof. Of these, an oligo-dT primer and a random primer tend to more easily anneal to any RNA than a primer specific to a particular sequence, and are likely to induce the synthesis of DNA from rRNA. Even when such an oligo-dT primer or random primer is used, the present invention can effectively suppress the synthesis of DNA from rRNA. From this viewpoint, the present invention is beneficial in performing reverse transcription reaction by using an oligo-dT primer and/or a random primer, and is particularly beneficial in performing reverse transcription reaction by using a combination of an oligo-dT primer and a random primer. The molar ratio of the oligo-dT primer to the random primer is in the range of, for example, 1:5 to 1:15, and preferably 1:8 to 1:12.

Examples of random primers include completely random primers and NSR (not-so-random) primers.

The completely random primers refer to a mixture of primers of various base sequences, in which each base sequence is completely random. The completely random primers may contain a sequence that is completely identical (or completely complementary) to the sequence of rRNA. Examples of completely random primers include completely random pentamers, completely random hexamers, completely random heptamers, completely random octamers, and combinations thereof. For example, completely random hexamers may be a mixture of all possible base sequences formed by the four nucleotides (A, T, C, and G) (4⁶ types).

NSR primers refer to primers prepared by removing a primer whose sequence is completely complementary to the sequence of rRNA from completely random primers. Examples of the sequence of rRNA to be removed include the sequence of 18S rRNA, the sequence of 28S rRNA, the sequence of 12S rRNA, the sequence of 16S rRNA, and combinations thereof.

Examples of NSR primers include those prepared by removing a hexamer with a sequence completely complementary to the sequence of rRNA from completely random hexamers. Those prepared by removing a sequence completely complementary to the sequence of rRNA from a primer set such as of completely random pentamers, completely random heptamers, and completely random octamers are also usable as NSR primers.

The use of such NSR primers enables the analysis of mRNA or the like with increased sensitivity.

The details of NSR primers are disclosed, for example, in 1) Amour et al., Digital transcriptome profiling using selective hexamer priming for cDNA synthesis, Nature Methods, Vol. 6, No. 9, 2009, pp. 647-649, 2) Ozsolak et al., Digital transcriptome profiling from attomole-level RNA samples, Genome Research, Vol. 20, 2010, pp. 519-525, and 3) U.S. Patent Application Publication No. 2010/0029511, the entire disclosures of which are incorporated herein by reference.

Although NSR primers are designed to not be completely complementary to the base sequence of rRNA, some of the primers possibly bind to rRNA. As a result, a reverse transcription product possibly contains DNA derived from rRNA in addition to DNA derived from template RNA. However, the use of a polymer having at least one functional group selected from the group consisting of a sulfuric acid group, a sulfonic group, and salt-form groups thereof can suppress the synthesis of DNA derived from rRNA.

From the standpoint of annealing, the length of the primers is, for example, 5 bases or more, and preferably 6 bases or more. From the standpoint of synthesis, the length of the primers is, for example, 30 bases or less, preferably 25 bases or less, or more preferably 20 bases or less.

The primers in the reverse transcription reaction composition can be of any concentration. The concentration of the primers is, for example, 1 to 10 µM, preferably 2 to 6 µM, or more preferably 3 to 5 µM.

### (3) Deoxyribonucleotide

The deoxyribonucleotide is preferably deoxyribonucleoside triphosphate. Examples of deoxyribonucleoside triphosphate include deoxycytidine triphosphate (dCTP), deoxyguanosine triphosphate (dGTP), deoxyadenosine triphosphate (dATP), deoxythymidine triphosphate (dTTP), deoxyuridine triphosphate (dUTP), derivatives thereof, and combinations of two or more thereof. Of these, for example, a mixture of dCTP, dGTP, dATP, and dTTP, a mixture of dCTP, dGTP, dATP, and dUTP, and a mixture of dCTP, dGTP, dATP, dTTP, and dUTP are preferable.

### (4) Reverse Transcriptase

The reverse transcriptase refers to any protein (enzyme) that has reverse transcription activity (RNA-dependent DNA polymerase activity); the reverse transcriptase can be any reverse transcriptase, and is preferably a polymerase that has reverse transcriptase activity. The reverse transcriptase is preferably one that has low RNase H activity or no RNase H activity. Examples of reverse transcriptases include avian myeloblastosis virus reverse transcriptase (AMV-RT), Moloney murine leukemia virus reverse transcriptase (MMLV-RT), human immunovirus reverse transcriptase (HIV-RT), EIRV-RT, RAV2-RT, C. *hydrogenoformans* DNA polymerase, rTthDNA polymerase, SuperScript I, SuperScript II, variants thereof, and derivatives thereof. Of these, MMLV-RT is preferable.

### (5) DNA Polymerase

The reverse transcription reaction composition may contain the following DNA polymerases or contain no such DNA polymerases: Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT (trademark), DEEP VENT (trademark), and variants thereof.

### (6) RNase Inhibitor

The reverse transcription composition may contain an RNase inhibitor. The RNase inhibitor can be any RNase inhibitor. Examples of RNase inhibitors include human placenta-derived proteins, rat lung-derived proteins, and swine liver-derived proteins.

### (7) Additives

The reverse transcription reaction composition may contain additives. Examples of additives include buffers, salts, and combinations of two or more thereof.

Examples of buffers include Tris, Tricine, Bis-Tricine, Hepes, Mops, Tes, Taps, Pipes, Caps, and mixtures of two or more thereof. The buffer is typically dissolved in water (preferably nuclease-free water) and used in the form of an aqueous solution.

Examples of salts include chlorides (e.g., lithium chloride, sodium chloride, potassium chloride, magnesium chloride, and manganese chloride), acetates (e.g., lithium acetate, sodium acetate, potassium acetate, magnesium acetate, and manganese acetate), and sulfates (e.g., potassium sulfate, magnesium sulfate, and manganese sulfate), and combinations of two or more thereof.

### 2-2. Incubation

The conditions for incubating the reverse transcription reaction composition are not particularly limited as long as the reverse transcription of template RNA proceeds, and any conditions known in the art can be applied. The temperature of incubation is, for example, 30 to 65°C, and preferably 35 to 60°C. The incubation time is, for example, 5 to 120 minutes, and preferably 10 to 60 minutes.

### 2-3. A Case in which Reverse Transcription of Template RNA is Performed by Reverse Transcription Amplification to Amplify cDNA by Using RNA as a Template (Also Called "RT-RamDA Method")

The RT-RamDA method refers to a nucleic acid amplification method that includes the step of incubating a mixture that contains template RNA, a primer, a degrading enzyme specific to a DNA strand in an RNA:DNA hybrid strand, an RNase H minus reverse transcriptase, and a substrate. In the RT-RamDA method, due to RNA-dependent DNA polymerase activity of the RNase H minus reverse transcriptase, complementary strand DNA (cDNA) of the template RNA is synthesized, and the cDNA strand of the hybrid strand of RNA and cDNA is randomly cleaved by the degrading enzyme specific to a DNA strand in an RNA:DNA hybrid strand. With the cleavage site as a starting point, the cDNA strand at the 3' side is peeled off from RNA due to strand displacement activity of the RNase H minus reverse transcriptase, and a new cDNA strand is synthesized at the portion peeled off by the RNase H minus reverse transcriptase. The details of the RT-RamDA method are disclosed, for example, in US Patent Application Publication No. 2017/0275685, the entire disclosure of which is incorporated herein by reference.

When reverse transcription of template RNA is performed by the RT-RamDA method, the reverse transcription reaction composition contains a degrading enzyme specific to a DNA strand in an RNA:DNA hybrid strand.

The degrading enzyme specific to a DNA strand in an RNA:DNA hybrid strand preferably has activity of cleaving the DNA strand in a hybrid strand of RNA and DNA. The enzyme for use can be, for example, a double strand-specific DNA degrading enzyme or a non-specific DNA degrading enzyme.

The double-strand-specific degrading enzyme (double-strand-specific nuclease; also referred to as DSN) for use may be a nuclease derived from a prokaryote or a eukaryote, and is preferably a double strand-specific DNA degrading enzyme derived from a crustacean or a modified product thereof. Specific examples include the following:
- *Solenocera melantho* (razor mud shrimp) DNase
- *Penaeus japonicus* (prawn) DNase
- *Paralithodes camtschaticus* (red king crab) DSN
- *Pandalus borealis* (Alaskan pink shrimp) dsDNase
- *Chionoecetes opilio* (snow crab) DSN
- Other DSN homologs

The double strand-specific DNA degrading enzyme preferably has DNA-degrading activity even at a temperature below 60°C. Of the DNA degrading enzymes above, double strand-specific DNA degrading enzymes derived from a prawn or shrimp or a modified product thereof are preferable.

The double strand-specific DNA degrading enzyme for use can be a commercially available product. Examples of commercially available products of double strand-specific DNA degrading enzymes include dsDNase (produced by ArcticZymes), Hl-dsDNase (produced by ArcticZymes), dsDNase (produced by Thermo Scientific), Shrimp DNase, Recombinant (produced by Affymetrix), Atlantis dsDNase (produced by Zymo Research), and Thermolabile Nuclease (produced by Roche).

The non-specific DNA degrading enzyme is, for example, an enzyme that has activity of cleaving the DNA strand of a hybrid strand of RNA and DNA, and substantially has no activity of cleaving the RNA strand of a hybrid strand of RNA and DNA and a single-stranded RNA, and preferably an enzyme that has relatively lower activity of cleaving a single-stranded DNA than the activity of cleaving the DNA strand of a hybrid strand of RNA and DNA. The non-specific DNA degrading enzyme preferably has DNA-degrading activity even at a temperature below 60°C. The non-specific DNA degrading enzyme for use may be a commercially available product. Examples of non-specific DNA degrading enzymes for use include DNase I (DNase I, produced by Thermo Fisher). The non-specific DNA degrading enzyme for use may be an enzyme derived from a prokaryote or a eukaryote, and is preferably a non-specific DNA degrading enzyme derived from a mammal or a modified product thereof, and more preferably a bovine-derived non-specific DNA degrading enzyme or a modified product thereof.

The "modified product" above refers to an enzyme obtained by modifying a naturally occurring amino acid sequence. Specifically, a modified product refers to an enzyme having an amino acid sequence that is at least 80% (preferably at least 90%, more preferably at least 95%) identical to a naturally occurring amino acid sequence, and an enzyme having an amino acid sequence that contains one or several (e.g., 1 to 10, preferably 1 to 5, more preferably 1 to 3) deletions, substitutions, and/or additions of amino acids as compared with a naturally occurring amino acid sequence.

When reverse transcription of template RNA is performed by the RT-RamDA method, the reverse transcription reaction composition may contain a single-stranded-DNA binding protein. The single-stranded-DNA binding protein is typically used together with a degrading enzyme specific to a DNA strand in an RNA:DNA hybrid strand. Examples of single-stranded-DNA binding proteins include T4 Gene 32 Protein, RecA, SSB (Single-Stranded DNA Binding Protein), and combinations of two or more thereof.

When reverse transcription of template RNA is performed by the RT-RamDA method, the reverse transcription reaction composition may be incubated under isothermal conditions or under thermal cycling conditions.

### (1) Isothermal Conditions

Incubation under isothermal conditions can be performed, for example, at a predetermined temperature of 25°C or higher and below 50°C, preferably at a predetermined temperature between 30 and 45°C, more preferably at a predetermined temperature between 35 and 40°C, for example, at 37°C for a predetermined period of time (e.g., 5 to 180 minutes, preferably 10 to 150 minutes).

Incubation at a predetermined temperature of 25°C or higher and below 50°C may be performed in two or more steps. For example, incubation can be performed at a predetermined temperature of 25°C or higher and below 30°C for 5 to 15 minutes, and then performed at a predetermined temperature of 30°C or higher and below 35°C for 5 to 15 minutes, followed by incubation at a predetermined temperature of 35°C or higher and below 50°C for a predetermined period of time (e.g., 5 to 60 minutes).

After incubation at a predetermined temperature of 25°C or higher and below 50°C, incubation may be performed, for example, at a predetermined temperature of 50°C or higher and below 100°C. The incubation at a predetermined temperature of 50°C or higher and below 100°C may be performed in two or more steps. For example, incubation may be performed at a predetermined temperature of 50°C or higher and below 80°C for 5 to 15 minutes, and then performed at a predetermined temperature between 80 and 90°C for 5 to 15 minutes.

### (2) Thermal Cycling Conditions

Incubation under thermal cycling conditions can be performed, for example, at predetermined temperature T1 that is 20°C or higher and below 30°C (e.g., 25°C) and at predetermined temperature T2 that is between 30 and 45°C (e.g., 37°C) in combination for a predetermined period of time for T1 (e.g., 1 to 3 minutes; for example, 2 minutes) and for a predetermined period of time for T2 (e.g., 1 to 3 minutes; for example, 2 minutes). This operation can be taken as one cycle and may be performed for preferably 10 to 40 cycles, and more preferably 15 to 35 cycles. Before the thermal cycling, incubation may be performed, for example, at a predetermined temperature of 25°C or higher and below 30°C for a predetermined period of time (e.g., 5 to 15 minutes) and performed at a predetermined temperature of 30°C or higher and below 35°C for a predetermined period of time (e.g., 5 to 15 minutes), followed by incubation at a predetermined temperature of 35°C or higher and below 50°C for a predetermined period of time (e.g., 1 to 5 minutes). After the above thermal cycling, incubation may be performed, for example, at a predetermined temperature of 50°C or higher and below 80°C for a predetermined period of time (e.g., 5 to 15 minutes) and then performed at a predetermined temperature between 80 and 90°C for a predetermined period of time (e.g., 5 to 15 minutes).

### 2-4. A Case in Which Reverse Transcription of Template RNA is Performed by RT-PCR

When reverse transcription of template RNA is performed by an RT-PCR method, the reverse transcription reaction composition can be incubated under the conditions described in section 2-2 above. Thereafter, the reverse transcriptase may be optionally inactivated. The method for inactivating reverse transcription is not particularly limited, and may be, for example, a method for performing incubation at 90 to 100°C for a predetermined period of time (e.g., 1 to 10 minutes).

When reverse transcription of template RNA is performed by an RT-PCR method, the method of synthesizing DNA by reverse transcription of template RNA includes the step of amplifying DNA contained in the incubated reverse transcription reaction composition (which is also referred to as a "reverse transcription product"). The step of amplifying DNA typically includes the step of incubating a composition for DNA amplification under thermal cycling conditions.

The composition for DNA amplification may contain a reverse transcription product as is, or may contain a reverse transcription product diluted with water, preferably nuclease-free water. For example, the reverse transcription product may be diluted so that the mass is 1/20 to 1/30.

The composition for DNA amplification contains, for example, a primer, a deoxyribonucleotide, and a DNA polymerase in addition to the reverse transcription product. When the reverse transcription reaction composition described in section 2-1 above contains components necessary for DNA amplification such as a DNA polymerase, the reverse transcription reaction composition can serve as a composition for DNA amplification as is, and both compositions are interchangeably referred to.

The primer is preferably a primer specific to DNA (including DNA that is reverse-transcribed from RNA) (a forward primer and a reverse primer).

The deoxyribonucleotide and DNA polymerase for use may be those described in sections 2-1 (3) and (5).

The composition for DNA amplification may contain an anti-DNA polymerase antibody, a reaction buffer, a metal ion (e.g., magnesium ion), a fluorescent dye, a fluorescently labeled probe, or a combination of two or more thereof.

Incubation under thermal cycling conditions may be performed, for example, at a predetermined temperature of 80°C or higher and below 100°C for a predetermined period of time (e.g., 10 to 30 seconds) and performed at a predetermined temperature between 50 and 70°C for a predetermined period of time (e.g., 30 seconds to 2 minutes). This operation can be taken as one cycle and may be performed preferably 10 to 50 cycles, and more preferably 15 to 40 cycles.

### 3. Method for Suppressing the Synthesis of DNA Derived from rRNA by Using a Polymer Having at Least one Functional Group Selected from the Group Consisting of a Sulfuric Acid Group, a Sulfonic Group, and Salt-form Groups Thereof

The method for suppressing the synthesis of DNA derived from rRNA by using a polymer having at least one functional group selected from the group consisting of a sulfuric acid group, a sulfonic group, and salt-form groups thereof typically includes the step of reverse-transcribing template RNA in the presence of the above polymer in a concentration of 10 pg/µL or more. This step enables template RNA to be specifically reverse-transcribed and enables DNA derived from template RNA to be specifically synthesized, thus suppressing the synthesis of DNA derived from rRNA. rRNA is preferably 18S rRNA, 28S rRNA, 12S rRNA, 16S rRNA, or a combination of two or more thereof. The conditions for reverse transcription are as described in sections 2-1 to 2-4 above.

### Compositions for Suppressing the Reverse Transcription of rRNA

The composition for suppressing the reverse transcription of rRNA preferably contains the polymer having at least one functional group selected from the group consisting of a sulfuric acid group, a sulfonic group, and salt-form groups thereof. The reverse transcription of rRNA cannot be limited as long as it is reverse transcription of an RNA sample that contains at least rRNA, and may be reverse transcription of an RNA sample that contains template RNA and rRNA. The RNA sample that contains template RNA and rRNA may be RNA extracted from cells or tissue, RNA that has been extracted from cells or tissue and then further purified, or like RNA, and is preferably RNA extracted from cells. This RNA for use may be the RNA described in section 2-1 (1) above.

The conditions for reverse transcription to be applied may be, for example, the same as the conditions described in sections 2-2 to 2-4 above.

The composition for suppressing the reverse transcription of rRNA may contain components as described in sections 2-1, 2-2, and 2-3 above (in particular, components necessary for reverse transcription) in addition to the above polymer.

The composition for suppressing the reverse transcription of rRNA may be in the form of a mixture of those components or in the form of a kit that contains such components separately.

### Reverse Transcription Reaction Composition for Preparing a Reverse Transcription Reaction Product for Use in Next-generation Sequencing

The reverse transcription reaction composition for preparing a reverse transcription reaction product for use in next-generation sequencing (NGS analysis: next-generation sequencing, also referred to as "next-generation sequencer analysis") preferably contains a polymer having at least one functional group selected from the group consisting of a sulfuric acid group, a sulfonic group, and salt-form groups thereof. The next-generation sequencing typically refers to a sequencing technology capable of simultaneously performing tremendous sequencing reactions of millions to billions. Examples of next-generation sequencing include a method for performing sequence analysis in parallel by using an amplification technique such as emulsion PCR and bridge PCR and a high-sensitivity detection technique such as single-molecule observation. The device for performing next-generation sequencing (sequencer) is not limited, and examples include MiSeq, HiSeq, NovaSeq (produced by Illumina, Inc.), Genetic Analyzer V2.0, Ion Proton (produced by Thermo Fisher Scientific), MinION, and PromethION (produced Nanopore). Next-generation sequencing is disclosed in, for example, U.S. Patent Application Publication No. 2014/178438, the entire disclosure of which is incorporated herein by reference.

The present invention can effectively reduce the reverse transcription reaction product from rRNA that could be noise in large-scale gene expression analysis, for example, in next-generation sequencing. In NGS analysis, because the number of reads obtained in one-time analysis is often limited, it is important to reduce unnecessary reads of DNA derived from rRNA. The present invention is advantageous in terms of costs because the present invention can reduce reads of DNA derived from rRNA that is not intended for analysis, is excellent in increasing the number of useful reads (the number of reads excluding the number of reads of rRNA: gene information amount), and would further reduce the number of times for performing NGS analysis. Thus, the reverse transcription reaction product prepared from an RNA sample that could contain rRNA in accordance with the present invention is suitable for use in next-generation sequencing. The RNA sample that could contain rRNA may be an RNA sample that contains template RNA and rRNA. The RNA sample that contains template RNA and rRNA may be RNA extracted from cells or tissue, RNA that has been extracted from cells or tissue and then further purified, or like RNA, and is preferably RNA extracted from cells. This RNA for use can be the same as the RNA described in section 2-1 (1).

The conditions for reverse transcription can be the same as the conditions described in, for example, sections 2-2 to 2-4 above.

The reverse transcription reaction composition for preparing a reverse transcription reaction product for use in next-generation sequencing may contain components as described in sections 2-1, 2-2, and 2-3 above (in particular, components necessary for reverse transcription) in addition to the above polymer.

The reverse transcription reaction composition for preparing a reverse transcription reaction product for use in next-generation sequencing may be in the form of a mixture of components or in the form of a kit that contains components separately.

### Examples

The present invention is described below in more detail with reference to Examples. However, the present invention is not limited to these Examples.

### Example 1: Evaluation 1 of Suppression of Synthesis of DNA Derived from rRNA by Heparin, Chondroitin Sulfate, or Hyaluronic Acid in RT-PCR method

In this Example, the following test was performed in order to examine the amount of ribosomal RNA-derived DNA synthesized in the presence of heparin sodium (glycosaminoglycan having a sulfuric acid salt group), sodium chondroitin sulfate C (glycosaminoglycan having a sulfuric acid salt group), or sodium hyaluronate (glycosaminoglycan having none of the sulfuric acid group, sulfonic group, and salt-form groups thereof) in the RT-PCR method.

No polymer was added or each polymer was added to the sample solution at a predetermined concentration in the range of 2 pg/µL to 2 ng/µL, and single-stranded cDNA was synthesized by a reverse transcription reaction. Thereafter, the effect of addition of each polymer on the amount of ribosomal RNA-derived DNA synthesized was analyzed by a quantitative polymerase chain reaction (qPCR, real-time PCR). Specifically, the analysis (wherein n = 3) was performed by the following method.

In this Example, 1 ng RNA purified by an RNeasy Mini Kit (produced by Qiagen) from HeLa S3 cells was used as a nucleic acid fragment sample.

Table 1 shows the components contained in the sample solution used in this Example for the reverse transcription reaction of the RNA (10 pg) and their final concentrations in the sample solution. The NSR primer (hexamer) was synthesized through the Sigma-Aldrich custom oligo service, then mixed to have the primer composition disclosed in Ozsolak et al., Digital transcriptome profiling from attomole-level RNA samples, Genome Research, Vol. 20, 2010, pp. 519-525, and used.

**Table 1**

| Final concentration | Component |
|---|---|
| 100 mM | Tris-HCl (pH8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0.4 µM | Oligo (dT)18 primer |
| 4 µM | NSR primer (hexamer) |
| 1 U/ µL | Reverse transcriptase (ReverTra Ace, produced by Toyobo Co., Ltd.) |
| 0.5 U/ µL | RNase inhibitor (RNase Inhibitor, produced by Toyobo Co., Ltd.) |
| 0,2 pg/ µL, 20 pg/ µL, 200 pg/ µL, or 2 ng/ µL | Each polymer (heparin sodium (produced by Nacalai Tesque, Inc.), sodium chondroitin sulfate C (produced by Nacalai Tesque, Inc.), sodium hyaluronate (produced by Toa Kasei Co., Ltd.)) |

Using sample solutions prepared by adding purified RNA to the above formulations, a reverse transcription reaction was performed. This reaction was performed at 37°C for 30 minutes, subsequently at 50°C for 5 minutes, and then at 95°C for 5 minutes to synthesize single-stranded cDNA.

The amounts of DNA synthesized were then compared by the following quantitative polymerase chain reaction (qPCR).

Each reverse transcription reaction solution was diluted with nuclease-free water (produced by Qiagen), and a 1/25 amount was used for the qPCR reaction. The qPCR was performed by using StepOnePlus (produced by Life Technologies) under the following conditions.

A qPCR reaction solution (20 µl (THUNDERBIRD (trademark) SYBR qPCR Mix (produced by Toyobo Co., Ltd.), 6 pmol of a forward primer, 6 pmol of a reverse primer, 2 µl of the diluted reverse transcription reaction solution, and nuclease-free water) was treated at 95°C for 1 minute for enzyme activation, and then subjected to denaturation at 95°C for 15 seconds and an extension reaction at 60°C for 1 minute. This operation consisting of the denaturation and the extension reaction was taken as one cycle and performed 40 cycles. The qPCR was performed by using a CFX96 real-time PCR analysis system, and the threshold was set at 100 RFU.

Melting curve analysis was performed at 95°C for 15 seconds, at 60°C for 15 seconds, and at 95°C for 15 seconds.

ACTB, which is messenger RNA (mRNA), and RNA18S, which is 18S ribosomal RNA, were used as target genes.

The primers for each gene are as follows.
ACTB (mRNA)
   Forward primer: CGCGAGAAGATGACCCAGAT (SEQ ID NO: 1)
   Reverse primer: GCCAGAGGCGTACAGGGATA (SEQ ID NO: 2)
RNA18S (ribosomal RNA)
   Forward primer: GATGGTAGTCGCCGTGCC (SEQ ID NO: 3)
   Reverse primer: GCCTGCTGCCTTCCTTGG (SEQ ID NO: 4)

Using the Ct values obtained as the analysis results, the average Ct value obtained without adding any polymers was subtracted from the average Ct value obtained with the addition of each polymer at various concentrations to calculate the ΔCt value (i.e., the value obtained with the addition of each polymer - the value obtained without adding any polymer). Table 2 shows the results of ACTB. Table 3 shows the results of RNA18S. Further, the ΔCt value of ACTB was subtracted from the ΔCt value of RNA18S to calculate a ΔΔCt value, and whether the inhibition of reverse transcription was specific to RNA18S was evaluated. Table 4 shows the results.

**Table 2**

| | Δ Ct value of ACTB | | |
|---|---|---|---|
| Polymer concentration | Sodium heparin | Sodium chondroitin sulfate | Sodium hyaluronate |
| 2 pg/*µ*L | 0.28 | 0.31 | 0.01 |
| 20 pg/*µ*L | 0.35 | 0.05 | -0.16 |
| 200 pg/*µ*L | 4.80 | 0.18 | -0.26 |
| 2 ng/*µ*L | 10.65 | 0.15 | -0.12 |

**Table 3**

| | Δ Ct value of RNA18S | | |
|---|---|---|---|
| Polymer concentration | Sodium heparin | Sodium chondroitin sulfate | Sodium hyaluronate |
| 2 pg/µL | 0.18 | 0.20 | 0.43 |
| 20 pg /µL | 0.96 | 0.56 | -0.15 |
| 200 pg/µL | 6.96 | 0.83 | 0.42 |
| 2 ng/µL | 12.74 | 0.90 | -0.11 |

**Table 4**

| | Δ Δ Ct value ( Δ Ct value of RNA18S - Δ Ct value of ACTB) | | |
|---|---|---|---|
| Polymer concentration | Sodium heparin | Sodium chondroitin sulfate | Sodium hyaluronate |
| 2 pg/µL | -0.10 | -0.11 | 0.43 |
| 20 pg/µL | 0.61 | 0.51 | 0.01 |
| 200 pg/µL | 2.16 | 0.65 | 0.69 |
| 2 ng/µL | 2.09 | 0.75 | 0.02 |

When sodium heparin or sodium chondroitin sulfate was added in a specific amount or more, the ΔCt value of RNA18S, which is ribosomal RNA, became greater than the ΔCt value of ACTB, which is mRNA, almost in proportion to the amount of sodium heparin or sodium chondroitin sulfate added. However, this effect was not observed when the amount of sodium heparin or sodium chondroitin sulfate added was as small as 2 pg/µL. These results confirmed that the addition of a specific amount or more of sodium heparin or sodium chondroitin sulfate can reduce the synthesized amount of DNA derived from ribosomal RNA. Further, when sodium hyaluronate was used, no concentration-dependent changes in ΔCt value or ΔΔCt value were observed.

### Example 2: Evaluation 2 of Suppression of rRNA-derived DNA Synthesis by Heparin in RT-PCR Method

In this Example, the following test was performed in order to confirm whether the reduction of the synthesized amount of ribosomal RNA-derived DNA by a polymer having at least one functional group selected from the group consisting of a sulfuric acid group, a sulfonic group, or salt-form groups thereof occurs even when a random primer is used.

No polymer was added or each polymer was added to the sample solution at a concentration of either 2 pg/µL or 20 pg/µL. Single-stranded cDNA was synthesized by a reverse transcription reaction. Thereafter, the amount of ribosomal RNA-derived DNA synthesized by adding each polymer was analyzed by a quantitative polymerase chain reaction (qPCR, real-time PCR). Specifically, the analysis wherein n = 2 was performed by the following method.

1 ng RNA purified by an RNeasy Mini Kit (produced by Qiagen) from HeLa S3 cells was used as a nucleic acid fragment sample in this Example.

Table 5 shows the components contained in the sample solution used in this Example for the reverse transcription reaction of the RNA (10 pg) and their final concentrations in the sample solution.

**Table 5**

| Final concentration | Component |
|---|---|
| 100mM | Tris-HCl (pH8.0) |
| 10mM | Magnesium chloride |
| 50mM | Potassium chloride |
| 1mM | dATP |
| 1mM | dTTP |
| 1mM | dGTP |
| 1mM | dCTP |
| 0.4µM | Oligo (dT)18 primer |
| 4 µM | Random primer (Hexamer) |
| 1U/µL | Reverse transcriptase (ReverTra Ace, produced by Toyobo Co., Ltd.) |
| 0.5U/µL | RNase inhibitor (RNase Inhibitor, produced by Toyobo Co., Ltd.) |
| 0 pg/µL, 2 pg/µL, or 20 pg/µL | Sodium heparin (Nacalai Tesque, Inc.) |

Using sample solutions prepared by adding the purified RNA to the compositions described above, a reverse transcription reaction was performed. This reaction was performed at 37°C for 30 minutes, subsequently at 50°C for 5 minutes, and then at 95°C for 5 minutes to synthesize single-stranded cDNA.

The amounts of DNA were then compared by the following quantitative polymerase chain reaction (qPCR).

Each reverse transcription reaction solution was diluted with nuclease-free water (produced by Qiagen), and a 1/25 amount was used for the qPCR reaction. The qPCR was performed by using StepOnePlus (produced by Life Technologies) under the following conditions.

A qPCR reaction solution (20 µl (THUNDERBIRD (trademark) SYBR qPCR Mix (produced by Toyobo Co., Ltd.), 6 pmol of a forward primer, 6 pmol of a reverse primer, 2 µl of the diluted reverse transcription reaction solution, and nuclease-free water) was treated at 95°C for 1 minute for enzyme activation and then subjected to denaturation at 95°C for 15 seconds and an extension reaction at 60°C for 1 minute. This operation consisting of the denaturation and the extension reaction was taken as one cycle and performed 40 cycles. The qPCR was performed by using a CFX96 real-time PCR analysis system, and the threshold was set at 100 RFU.

Melting curve analysis was performed at 95°C for 15 seconds, at 60°C for 15 seconds, and at 95°C for 15 seconds.

ACTB, which is messenger RNA (mRNA), and RNA18S, which is 18S ribosomal RNA, were used as target genes.

The primers for the genes were the same as those used in Example 1.

Using the Ct values obtained as the analysis results, the average Ct value obtained without adding any polymer was subtracted from the average Ct value obtained with the addition of each polymer at various concentrations to calculate the ΔCt value (i.e., the value obtained with the addition of each polymer - the value obtained without adding any polymer). Table 6 shows the results of ACTB. Table 7 shows the results of RNA18S. Further, the ΔCt value of ACTB was subtracted from the ΔCt value of RNA18S to calculate a ΔΔCt value, and whether the inhibition of reverse transcription was specific to RNA18S was evaluated. Table 8 shows the results.

**Table 6**

| Sodium heparin concentration | Δ Ct value of ACTB |
|---|---|
| 2 pg/µL | 0.11 |
| 20 pg/µL | 0.39 |

**Table 7**

| Sodium heparin concentration | Δ Ct value of RNA18S |
|---|---|
| 2 pg/µL | 0.10 |
| 20 pg/µL | 0.90 |

**Table 8**

| Sodium heparin concentration | Δ Δ Ct value (Δ Ct value of RNA18S - Δ Ct value of ACTB) |
|---|---|
| 2 pg/µL | -0.01 |
| 20 pg/µL | 0.52 |

Even when a random primer was used, the presence of 20 pg/µL of sodium heparin with the random primer made the ΔCt value of RNA18S, which is a ribosomal RNA, greater than the ΔCt value of ACTB, which is an mRNA. This result confirmed that adding a specific amount or more of sodium heparin can reduce the synthesized amount of DNA derived from ribosomal RNA.

### Example 3: Evaluation 3 of Suppression of Synthesis of DNA Derived from rRNA by Chondroitin Sulfate or Hyaluronic Acid in RT-PCR Method

In this Example, the following test was performed in order to confirm whether the effect obtained by using a random primer in Example 2 can also be obtained by using other polymers. In this Test Example, sodium chondroitin sulfate (sodium chondroitin sulfate C) was used as a glycosaminoglycan having a sulfuric acid salt group. For comparison, sodium hyaluronate was used as a glycosaminoglycan having none of the sulfuric acid group, sulfonic group, and salt-form groups thereof. No polymer was added or each polymer was added to the sample solution at a concentration of either 50 ng/µL or 800 ng/µL, and single-stranded cDNA was synthesized by reverse transcription reaction. Thereafter, the amount of ribosomal RNA-derived DNA synthesized by adding each polymer was analyzed by a quantitative polymerase chain reaction (qPCR, real-time PCR). Specifically, the analysis wherein n = 2 was performed by the following method.

In this Example, 1 ng RNA purified by an RNeasy Mini Kit (produced by Qiagen) from HeLa S3 cells was used as a nucleic acid fragment sample.

Table 9 shows the components contained in the sample solution used in this Example for the reverse transcription reaction of the RNA (10 pg) and their final concentrations in the sample solution.

**Table 9**

| Final concentration | Component |
|---|---|
| 100 mM | Tris-HCl (pH8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0. 4 µM | Oligo (dT) 18 primer |
| 4 µM | Random primer (Hexamer) |
| 1 U/µL | Reverse transcriptase (ReverTra Ace, produced by Toyobo Co., Ltd.) |
| 0. 5 U/µL | RNase inhibitor (RNase Inhibitor, produced by Toyobo Co., Ltd.) |
| 50 ng/µL or 800 ng/ µL | Each polymer (sodium chondroitin sulfate C (produced by Nacalai Tesque, Inc.), sodium hyaluronate (produced by Toa Kasei Co., Ltd.) |

Using sample solutions prepared by adding purified RNA to the above formulations, a reverse transcription reaction was performed. This reaction was performed at 37°C for 30 minutes, subsequently at 50°C for 5 minutes, and then at 95°C for 5 minutes to synthesize single-stranded cDNA.

The amounts of DNA were then compared by the following quantitative polymerase chain reaction (qPCR).

Each reverse transcription reaction solution was diluted with nuclease-free water (produced by Qiagen), and a 1/25 amount was used for the qPCR reaction. The qPCR was performed by using StepOnePlus (produced by Life Technologies) under the following conditions.

A qPCR reaction solution (20 µl (THUNDERBIRD (trademark) SYBR qPCR Mix (produced by Toyobo Co., Ltd.), 6 pmol of a forward primer, 6 pmol of a reverse primer, 2 µl of the diluted reverse transcription reaction solution, and nuclease-free water) was treated at 95°C for 1 minute for enzyme activation and then subjected to denaturation at 95°C for 15 seconds and an extension reaction at 60°C for 1 minute. This operation consisting of the denaturation and the extension reaction was taken as one cycle and performed 40 cycles. The qPCR was performed by using a CFX96 real-time PCR analysis system, and the threshold was set at 100 RFU.

Melting curve analysis was performed at 95°C for 15 seconds, at 60°C for 15 seconds, and at 95°C for 15 seconds.

ACTB, which is messenger RNA (mRNA), and RNA18S, which is 18S ribosomal RNA, were used as target genes.

The primers for each gene were the same as those used in Example 1.

Using the Ct values obtained as the analysis results, the average Ct value obtained without adding any polymer was subtracted from the average Ct value obtained with the addition of each polymer at various concentrations to calculate the ΔCt value (i.e., the value obtained with the addition of each polymer - the value obtained without adding any polymer). Table 10 shows the results of ACTB. Table 11 shows the results of RNA18S. Further, the ΔCt value of ACTB was subtracted from the ΔCt value of RNA18S to calculate a ΔΔCt value, and whether the inhibition of reverse transcription was specific to RNA18S was evaluated. Table 12 shows the results.

**Table 10**

| | Δ Ct value of ACTB | |
|---|---|---|
| Polymer concentration | Sodium chondroitin sulfate | Sodium hyaluronate |
| 50 ng/µL | 0. 16 | 0. 07 |
| 800ng/µL | 0. 8 6 | -0.09 |

**Table 11**

| | Δ Ct value of RNA18S | |
|---|---|---|
| Polymer concentration | Sodium chondroitin sulfate | Sodium hyaluronate |
| 50 ng/µL | 1. 35 | 0. 14 |
| 800ng/µL | 3. 7 9 | -0.11 |

**Table 12**

| | Δ ΔCt value (Δ Ct value of RNA18S - Δ Ct value of ACTB) | |
|---|---|---|
| Polymer concentration | Sodium chondroitin sulfate | Sodium hyaluronate |
| 50ng/*µ*L | 1. 19 | 0. 0 7 |
| 800ng/µL | 2. 93 | -0. 0 2 |

Results similar to those of Example 2 were obtained. Specifically, even when a random primer was used, the ΔCt value of RNA18S, which is a ribosomal RNA, became greater than the ΔCt value of ACTB, which is an mRNA, in proportion to the amount of sodium chondroitin sulfate added. This result confirmed that the addition of a specific amount or more of sodium chondroitin sulfate can reduce the amount of DNA derived from ribosomal RNA. In contrast, when sodium hyaluronate containing none of the sulfuric acid group, sulfonic group, or salt-form groups thereof was added, no significant changes in ΔCt value or ΔΔCt value were observed even at a final concentration of 800 ng/µL.

### Example 4: Evaluation of Suppression of Synthesis of DNA Derived from rRNA by Polyvinyl Sulfonic Acid in RT-PCR Method

In this Example, the following test was performed in order to examine the amount of ribosomal RNA-derived DNA synthesized in the presence of sodium polyvinyl sulfonate (PVSA) (a polymer having a sulfonic acid salt group).

No PVSA was added or PVSA was added to the sample solution at a concentration in the range of 20 pg/µL to 20 ng/µL, and single-stranded cDNA was synthesized by reverse transcription reaction. Thereafter, the amounts of ribosomal RNA-derived DNA synthesized in the presence of the polymer at various concentrations was analyzed by a quantitative polymerase chain reaction (qPCR, real-time PCR). Specifically, the analysis (wherein n = 3) was performed by the following method.

In this Example, 1 ng RNA purified by an RNeasy Mini Kit (produced by Qiagen) from HeLa S3 cells was used as a nucleic acid fragment sample. The NSR primer (hexamer) was synthesized through the Sigma-Aldrich custom oligo service, then mixed to have the primer composition disclosed in Ozsolak et al., Digital transcriptome profiling from attomole-level RNA samples, Genome Research, Vol. 20, 2010, pp. 519-525, and used. The volume of the reaction solution was set to 9 µL.

Table 13 shows the components of the sample solution used in this Example for the reverse transcription reaction of the RNA (10 pg) and their final concentrations in the sample solution.

**Table 13**

| Final concentration | Component |
|---|---|
| 100mM | Tris-HCl (pH8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0. 4 µM | Oligo (dT)18 primer |
| 4 µM | Random primer or NSR primer (the primers are both hexamers) |
| 1 U/µL | Reverse transcriptase (ReverTra Ace, produced by Toyobo Co., Ltd.) |
| 0. 5U/µL | RNase inhibitor (RNase Inhibitor, produced by Toyobo Co., Ltd.) |
| 20 pg/µL, 200 pg/µL, 2 ng/µL, or 20 ng/µL | Sodium PVSA (produced by Sigma Corporation) |

Using sample solutions prepared by adding purified RNA to the above formulations, a reverse transcription reaction was performed. This reaction was performed at 37°C for 30 minutes, subsequently at 50°C for 5 minutes, and then at 95°C for 5 minutes to synthesize single-stranded cDNA.

Thereafter, the amounts of DNA were compared by the following quantitative polymerase chain reaction (qPCR).

Each reverse transcription reaction solution was diluted with nuclease-free water (produced by Qiagen), and a 1/25 amount was used for the qPCR reaction. The qPCR was performed by using StepOnePlus (produced by Life Technologies) under the following conditions.

A qPCR reaction solution (20 µl (THUNDERBIRD (trademark) SYBR qPCR Mix (produced by Toyobo Co., Ltd.), 6 pmol of a forward primer, 6 pmol of a reverse primer, 2 µl of the diluted reverse transcription reaction solution, and nuclease-free water) was treated at 95°C for 1 minute for enzyme activation and then subjected to denaturation at 95°C for 15 seconds and an extension reaction at 60°C for 1 minute. This operation consisting of the denaturation and the extension reaction was taken as one cycle and performed 40 cycles. The qPCR was performed by using a CFX96 real-time PCR analysis system, and the threshold was set at 100 RFU.

Melting curve analysis was performed at 95°C for 15 seconds, at 60°C for 15 seconds, and at 95°C for 15 seconds.

ACTB, which is messenger RNA (mRNA), and RNA18S, which is 18S ribosomal RNA, were used as target genes.

The primers for each gene were the same as those used in Example 1.

Using the Ct values obtained as the analysis results, the average Ct value obtained without adding any polymer was subtracted from the average Ct value obtained with the addition of each polymer at various concentrations to calculate the ΔCt value (i.e., the value obtained with the addition of each polymer - the value obtained without adding any polymer). Table 14 shows the results of ACTB. Table 15 shows the results of RNA18S. Further, the ΔCt value of ACTB was subtracted from the ΔCt value of RNA18S to calculate a ΔΔCt value, and whether the inhibition of reverse transcription was specific to RNA18S was evaluated. Table 16 shows the results.

**Table 14**

| | Δ Ct value of ACTB | |
|---|---|---|
| Polymer concentration | Sodium PVSA (random primer) | Sodium PVSA (NSR primer) |
| 20pg/*µ*L | 0. 2 5 | -0.03 |
| 200pg/µL | -0. 13 | 0. 68 |
| 2ng/*µ*L | 0. 93 | 9. 3 7 |
| 20ng/*µ*L | 4. 82 | 9. 4 7 |

**Table 15**

| | Δ Ct value of RNA18S | |
|---|---|---|
| Polymer concentration | Sodium PVSA (random primer) | Sodium PVSA (NSR primer) |
| 20pg/*µ*L | 0. 29 | 0. 2 3 |
| 200pg/µL | 1. 60 | 2. 6 5 |
| 2ng/*µ*L | 9. 81 | 11. 34 |
| 20ng/*µ*L | 15. 32 | 1 5. 02 |

**Table 16**

| | Δ Δ Ct value (Δ Ct value of RNA18S - Δ Ct value of ACTB) | |
|---|---|---|
| Polymer concentration | Sodium PVSA (random primer) | Sodium PVSA (NSR primer) |
| 20pg/µL | 0.04 | 0. 26 |
| 200pg/µL | 1. 7 2 | 1. 9 7 |
| 2ng/µL | 8. 88 | 1.98 |
| 20ng/µL | 10.50 | 5. 55 |

In proportion to the amount of sodium PVSA added, the ΔCt value of RNA18S, which is a ribosomal RNA, became greater than the ΔCt value of ACTB, which is an mRNA. This result was observed regardless of whether a random primer or an NSR primer was used. In particular, when the random primer was used, this tendency was strong. This result confirmed that the addition of a specific amount or more of sodium PVSA can reduce the synthesized amount of DNA derived from ribosomal RNA.

### Example 5: Inhibition of Synthesis of DNA Derived from Ribosomal RNA by Heparin in RT-RamDA Method

In this Example, the following test was performed in order to examine the amount of ribosomal RNA-derived DNA synthesized in the presence of sodium heparin in the RT-RamDA method.

Sodium heparin was added to the sample solution, and single-stranded cDNA was synthesized by the RT-RamDA method. A library was then prepared, and the ribosomal RNA ratio was calculated by NGS analysis. Specifically, the following method was used.

In this Example, 10 pg RNA purified by an RNeasy Mini Kit (produced by Qiagen) from NIH3T3 cells was used as a nucleic acid fragment sample.

Table 17 shows the components contained in the sample solution used in this Example for subjecting the RNA (10 pg) to the RT-RamDA method and their final concentrations in the sample solution. The NSR primer (hexamer) was synthesized through the Sigma-Aldrich custom oligo service, then mixed to have the primer composition disclosed in Ozsolak et al., Digital transcriptome profiling from attomole-level RNA samples, Genome Research, Vol. 20, 2010, pp. 519-525, and used. The volume of the reaction solution was set to 9 µL.

**Table 17**

| Final concentration | Component |
|---|---|
| 100mM | Tris-HCl (pH8. 0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0. 4µM | Oligo (dT)18 primer |
| 4 µM | NSR primer (hexamer) |
| 1 U/µL | Reverse transcriptase (ReverTra Ace, produced by Toyobo Co., Ltd.) |
| 0. 5 U/µL | RNase inhibitor (RNase Inhibitor, produced by Toyobo Co., Ltd.) |
| 0. 2 U/µL | DNase (DNase I, produced by Thermo Fisher Scientific Inc.) |
| 5 0 ng/µL | T4 Gene 32 Protein |
| 0 ng/µL, 0.1 ng/µL, or 1 ng/µL | Sodium heparin (Nacalai Tesque, Inc.) |

Using sample solutions prepared by adding purified RNA to the above formulations, the RT-RamDA method was performed. In this method, the reaction was performed at 25°C for 10 minutes, 30°C for 10 minutes, 37°C for 30 minutes, 50°C for 5 minutes, and 85°C for 5 minutes.

Thereafter, 9 µL of the obtained cDNA solution was used to produce double-stranded cDNA by using the 2nd strand synthesis buffer, the 2nd strand synthesis enzyme, and the 2nd strand synthesis primer mix of a GenNext^{®} RamDA-seq^{®} Single Cell Kit (produced by Toyobo Co., Ltd.). A library was then prepared by using Nextera (produced by Illumina K.K.), and NGS (next-generation sequencing) was performed by MiSeq (produced by Illumina) using a MiSeq Reagent Kit v3 (150 Cycles) (Illumina). The procedure was in accordance with the instruction manual. The analysis was performed by using an Illumina BaseSpace (produced by Illumina), and the proportion of ribosomal RNA in the reads was calculated. The procedure was according to the instruction manual. Table 18 shows the results.

**Table 18**

| | Number of Reads | % Total Aligned | % Abundant | % Abundant /% Total Aligned |
|---|---|---|---|---|
| No addition of sodium heparin | 741, 842 | 95. 0% | 45.3% | 47. 7% |
| 0.1 ng/µL sodium heparin added | 798, 147 | 94. 8% | 18.4% | 19.4% |
| 1 ng/µL sodium heparin added | 776, 206 | 91. 3% | 9.4% | 10. 3% |

In Table 18, the "Number of Reads" refers to the total number of reads obtained. The "% Total Aligned" refers to the percentage of reads that matched the reference genome (UCSC mm10) containing ribosomal RNA among the total reads. The "% Abundant" refers to the percentage of ribosomal RNA. The "% Abundant/% Total Aligned" indicates the percentage of ribosomal RNA in the reads that matched the reference genome.

When the condition in which sodium heparin was added was used, the "% Abundant/% Total Aligned" ratio was low and the proportion of ribosomal RNA was reduced, as compared to the condition in which no sodium heparin was added.

This result confirmed that the suppression of DNA synthesis from ribosomal RNA by a polymer having at least one functional group selected from the group consisting of a sulfuric acid group, a sulfonic group, and salt-form groups thereof, such as heparin sodium, can also be used in the RT-RamDA method and NGS analysis.

## Claims

1. A method for synthesizing DNA by reverse transcription of template RNA, the method comprising suppressing synthesis of DNA derived from ribosomal RNA using a polymer comprising at least one functional group selected from the group consisting of a sulfuric acid group, a sulfonic group, and salt-form groups thereof wherein the polymer is present in a concentration of 10 pg/µL or more in a reverse transcription reaction solution.

2. The method according to claim 1, wherein the polymer comprises at least one member selected from the group consisting of sulfated glycosaminoglycan, polyvinyl sulfonic acid, and salts thereof.

3. The method according to claim 1 or 2, wherein the polymer comprises at least one member selected from the group consisting of heparin, heparan sulfate, chondroitin sulfate, and salts thereof.

4. The method according to claim 1 or 2, wherein the reverse transcription reaction solution contains the polymer in a concentration of 10 pg/µL or more and 1 µg/µL or less.

5. The method according to claim 1 or 2, wherein the reverse transcription is performed by using a random primer.

6. The method according to claim 5, wherein the random primer has a base sequence that is not completely complementary to the base sequence of the ribosomal RNA.

7. The method according to claim 1 or 2, wherein the reverse transcription is performed in an RT-PCR method or RT-RamDA method.

8. The method according to claim 1 or 2, wherein the template RNA is RNA extracted from a cell.

9. A composition for suppressing reverse transcription of ribosomal RNA,
the composition comprising a polymer having at least one functional group selected from the group consisting of a sulfuric acid group, a sulfonic group, and salt-form groups thereof,
the composition being used in an amount such that a reverse transcription reaction solution contains the polymer in a concentration of 10 pg/µL or more.

10. A reverse transcription reaction composition for preparing a reverse transcription reaction product for use in next-generation sequencing, the reverse transcription reaction composition comprising a polymer having at least one functional group selected from the group consisting of a sulfuric acid group, a sulfonic group, and salt-form groups thereof in an amount of 10 pg/µL or more.

11. The composition according to claim 9 or 10, wherein the reverse transcription is performed in an RT-PCR method or an RT-RamDA method.

12. The composition according to claim 9 or 10, which is in the form of a kit.
